# EUROPEAN PATENT SPECIFICATION

(11) **EP 1 109 574 B1**
(45) Date of publication and mention of the grant of the patent: **16.04.2008**
(21) Application number: 99969034.0
(22) Date of filing: 08.09.1999
(51) Int. Cl.: A61K 39/02, A61K 39/245, A61K 39/12

(54) **VACCINE AGAINST SEXUALLY TRANSMITTED DISEASES**
IMPFSTOFF GEGEN GESCHLECHTLICH ÜBERTRAGENE KRANKHEITEN
VACCIN CONTRE LES MALADIES SEXUELLEMENT TRANSMISSIBLES

(30) Priority: 11.09.1998 GB 9819898
(43) Date of publication of application: 27.06.2001
(73) Proprietor: GlaxoSmithKline Biologicals s.a., 1330 Rixensart (BE)
(72) Inventor: SLAOUI, Moncef, Mohamed, B-1330 Rixensart (BE); VANDEPAPELIERE, Pierre G., B-1330 Rixensart (BE)
(74) Representative: Tyrrell, Arthur William Russell
(86) International application number: PCT/EP1999/006623
(87) International publication number: WO 2000/015255

(56) References cited:
- WO-A-92/16231
- WO-A-95/17209
- US-A- 5 776 468
- STRAUS S.E. ET AL: "Placebo-controlled trial of vaccination with recombinant glycoprotein D of herpes simplex virus type 2 for immunotherapy of genital herpes." LANCET, (1994) 343/8911 (1460-1463). , XP002128775
- THOELEN S. ET AL: "Safety and immunogenicity of a hepatitis B vaccine formulated with a novel adjuvant system" VACCINE, vol. 16, no. 7, April 1998 (1998-04), pages 708-714, XP002128776
- STRAUS S.E. ET AL: "Immunotherapy of recurrent genital herpes with recombinant herpex simplex virus type 2 glycoproteins D and B: results of a placebo-controlled vaccine" THE JOURNAL OF INFECTIOUS DISEASES, vol. 176, 1997, pages 1129-1134, XP000867468
- "Novel Herpes Vaccine in Development" DOCTOR'S GUIDE TO MEDICAL & OTHER NEWS, [Online] 10 August 1995 (1995-08-10), page 1-2 XP002128777 Retrieved from the Internet: <URL:http://www.pslgroup.com/dg/5cce.htm> [retrieved on 2000-01-20]
- ASHLEY R.L. ET AL: "Cervical antibody responses to a herpes simplex virus type 2 glycoprotein subunit vaccine." JOURNAL OF INFECTIOUS DISEASES, (1998) 178/1 (1-7). , XP000867506
- APICELLA M.A. ET AL: "Vaccines for sexually transmitted diseases." CURRENT OPINION IN INFECTIOUS DISEASES, (1996) 9/1 (52-55). , XP000867508
- L.R.STANBERRY ET AL.: "Glycoprotein-D-Adjuvant vaccine to prevent genital herpes." THE NEW ENGLAND JOURNAL OF MEDICINE, vol. 347, no. 21, 21 November 2002 (2002-11-21), pages 1652-1661,

## Description

The present invention relates to one or more antigens for the prevention of sexually transmitted diseases and the use thereof in the formulation of a vaccine, for administration to female human subjects, for the prevention of infections associated with pathogens which cause sexually transmitted diseases. The invention also relates to a method of administering the vaccine to females to prevent infections associated with pathogens which cause sexually transmitted diseases.

Pathogens which cause sexually transmitted diseases (STDs) are known and there is an urgent need for effective vaccines to prevent such conditions.

Sometimes sexually transmitted diseases are caused by one or more pathogens.
Combination vaccines, able to prevent one or more STDs are therefore also required.

Straus et al (Lancet 1994 343: 1460-1463) describes a placebo-controlled trial of vaccination with recombinant glycoprotein D of herpes simplex virus type 2 for immunotherapy of genital herpes.

Thoelen et al (Vaccine 1998 16: 708-714) describes the safety and immunogenicity of a hepatitis B vaccine formulated with a novel adjuvant system.

Straus et al (Journal of Infectious Diseases 1997 176: 1129-1134) describes immunotherapy of recurrent genital herpes with recombinant herpes simplex virus type 2 glycoproteins D and B, and provides the results of a placebo-controlled vaccine trial.

WO92/16231 describes a vaccine comprising HSV glycoprotein D or an immunological fragment thereof in conjunction with 3-O-deacylated monophosphoryl lipid A (3D-MPL) and a suitable carrier.

US 5,776,468 describes vaccine compositions comprising small particles of 3D-MPL, including a vaccine comprising an HSV glycoprotein D or an immunological fragment thereof.

The present invention provides the use of an HSV glycoprotein D or an immunological fragment thereof and an adjuvant in the preparation of a vaccine for administration to an HSV1-/2- human female subject for the prevention of genital herpes disease.

Preferably the adjuvant is a TH-1 inducing adjuvant.

Suitable adjuvants for use in the invention include those well known in the art of vaccine formulation. By 'TH-1 inducing adjuvant' is meant an adjuvant which is a preferential stimulator of TH1 cell response.

A recognised signal that a TH1 response has been stimulated is the enhanced production of TH1-type cytokines *eg*. IFN-γ and IL-2. IFN-γ secretion is associated with protective responses against intracellular pathogens, including parasites, bacteria and viruses. Activation of leucocytes by IFN-γ enhances killing of intracellular pathogens and increases expression of Fc receptors. Direct cytotoxicity may also occur, especially in synergism with lymphotoxin (another product of TH1 cells). IFN-γ is also both an inducer and a product of NK cells, which are major innate effectors of protection. TH1 type responses, either through IFN-γ or other mechanisms, provide preferential help for murine IgG2a immunoglobulin isotypes.

In contrast, TH-2 type responses are associated with humoral mechanisms and the secretion of IL-4, IL-5, IL-6, IL-10 and tumour necrosis factor-beta.

Adjuvants which are capable of preferential stimulation of the TH1 cell response are described in International Patent Application Nos. WO 94/00153 and WO 95/17209.

3 De-O-acylated monophosphoryl lipid A (3D-MPL) is one such adjuvant. This is known from GB 2220211 (Ribi). Chemically it is a mixture of 3 De-O-acylated monophosphoryl lipid A with 4, 5 or 6 acylated chains and is manufactured by Ribi Immunochem Montana. A preferred 'small particle' form of 3 De-O-acylated monophosphoryl lipid A is disclosed in EP O 689 454B1 (SmithKline Beecham Biologicals SA).

In such 'small particle' 3-DMPL the particles of 3D-MPL are small enough to be sterile filtered through a 0.22micron membrane (as described in European Patent number 0 689 454).

Another preferred adjuvant which may be used in the present invention comprises QS21, an Hplc purified non-toxic fraction derived from the bark of Quillaja Saponaria Molina. Optionally this may be admixed with 3 De-O-acylated monophosphoryl lipid A (3D-MPL), optionally together with an carrier.

The method of production of QS21 is disclosed (as QS21) in US patent No. 5,057,540 and is available from Aquilla Pharmaceuticals.

Non-reactogenic adjuvant formulations containing QS21 have been described previously (WO 96/33739). Such formulations comprising QS21 and cholesterol have been shown to be successful TH1 stimulating adjuvants when formulated together with an antigen. Thus vaccine compositions which form part of the present invention may include a combination of QS21 and cholesterol.

Further adjuvants which are preferential stimulators of TH1 cell response include immunomodulatory oligonucleotides, for example unmethylated CpG sequences as disclosed in WO 96/02555.

Combinations of different TH1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of 1 : 10 to 10 : 1; preferably 1:5 to 5 : 1 and often substantially 1 : 1. The preferred range for optimal synergy is 2.5 : 1 to 1 : 1 3D MPL: QS21.

Preferably a carrier is also present in the vaccine composition according to the invention. The carrier may be an oil in water emulsion, or an aluminium salt. Other mineral salts may also be used as a carrier such as salts of calcium, iron or zinc. Other carriers include polyphosphazene, liposomes and ISCOMS.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline. A preferred oil-in-water emulsion comprises a metabolisible oil, such as squalene, alpha tocopherol and Tween 80. Additionally the oil in water emulsion may contain span 85 and/or lecithin.

Typically for human administration QS21 and 3D MPL will be present in a vaccine in the range of 1µg - 500µg, such as 10-100µg, preferably 10µg - 50µg per dose. Typically the oil in water will comprise from 2 to 10% squalene, from 2 to 10% alpha tocopherol and from 0.3 to 3% tween 80. Preferably the ratio of squalene: alpha tocopherol is equal or less than 1 as this provides amore stable emulsion. Span 85 may also be present at a level of 1 %. In some cases it may be advantageous that the vaccines of the present invention will further contain a stabiliser.

A particularly potent adjuvant formulation involving QS21, 3D-MPL and tocopherol in an oil in water emulsion is described in WO 95/17210.

In a preferred aspect aluminium hydroxide (alum) or aluminium phosphate will be included in the vaccine composition which is used or manufactured according to the invention.

In a particularly preferred aspect the antigens in the vaccine composition used or manufactured according to the invention are combined with 3D-MPL and alum.

Vaccines employed in the present invention may, if desired, comprise adjuvant molecules of general formula (I):

HO(CH₂CH₂O)ₙ-A-R

wherein, n is 1-50, A is a bond or -C(O)-, R is C₁₋₅₀ alkyl or Phenyl C₁₋₅₀ alkyl.

One embodiment of the present invention consists of a vaccine formulation comprising a polyoxyethylene ether of general formula (I), wherein n is between 1 and 50, preferably 4-24, most preferably 9; the *R* component is C₁₋₅₀, preferably C₄-C₂₀ alkyl and most preferably C₁₂ alkyl, and A is a bond. The concentration of the polyoxyethylene ethers should be in the range 0.1-20%, preferably from 0.1-10%, and most preferably in the range 0.1-1 % . Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index (12^{th} ed: entry 7717).

HSV-2 is the primary etiological agent of herpes genitalis. HSV-1 is the causative agent of herpes labialis. Together, these viruses are characterised by their ability to induce both acute diseases and to establish a latent infection, primarily in neuronal ganglia cells.

WO 92/16231 provides further background information about genital herpes and describes a vaccine which can be used to treat people susceptible to HSV infections comprising HSV glycoprotein D or an immunological fragment thereof in conjunction with 3-O-deacylated monophosphoryl lipid A and a suitable carrier.

The specification of WO 92/16231 provides details of glycoprotein D, immunological fragments thereof, and 3-DMPL and methods for obtaining it. The specification describes some promising tests of a candidate vaccine in animal models but no data in humans are given.

In a preferred aspect the use according to the invention relates to the prevention of HSV-2 infections.

The vaccine which may be used in the present invention comprises glycoprotein D or an immunological fragment thereof which is typically from HSV-2.

Glycoprotein D is located on the viral membrane, and is also found in the cytoplasm of infected cells (Eisenberg R.J. et al; J of Virol 1980 35 428-435). It comprises 393 amino acids including a signal peptide and has a molecular weight of approximately 60 kD. Of all the HSV envelope glycoproteins this is probably the best characterised (Cohen et al J. Virology 60 157-166). In vivo it is known to play a central role in viral attachment to cell membranes. Moreover, glycoprotein D has been shown to be able to elicit neutralising antibodies in vivo (Eing et al J. Med. Virology 127: 59-65). However, latent HSV-2 virus can still be reactivated and induce recurrence of the disease despite the presence of high neutralising antibodies titre in the patients sera.

As described in WO 92/ 16231, a preferred embodiment thereof is a truncated HSV-2 glycoprotein D of 308 amino acids which comprises amino acids 1 through 306 of the naturally occurring glycoprotein with the addition of aparagine and glutamine at the C-terminal end of the truncated protein devoid of its membrane anchor region. This form of the protein includes the signal peptide which is cleaved to yield a mature 283 amino acid protein. The production of such a protein in Chinese Hamster Ovary cells has been described in EP - B- 139 417.

The mature truncate preferably used in the vaccine formulation within the scope of the invention may be designated recombinant gD2t (rgD2t) or simply (as hereinbelow) gD2t.

The HSV antigen may be chemically or otherwise conjugated to a particulate carrier as described in WO 92/16231.

In one preferred aspect the vaccine for use in the invention comprises gD2t, 3-DMPL (especially small particle 3-DMPL) and aluminium hydroxide (alum).

Combination vaccines adminstered or prepared according to the present invention will contain an immunoprotective quantity of the antigens and may be prepared and administered by conventional techniques.

Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of antigen in each vaccine dose is selected as an amount which induces an immunoprotective or therapeutic response without significant, adverse side effects in typical vaccinees. Such amount will vary depending upon which specific immunogen is employed. Generally, it is expected that each dose will comprise 1-1000 µg of protein, preferably 2-100 µg, most preferably 4-40 µg. An optimal amount for a particular vaccine can be ascertained by standard studies involving observation of antibody titres and other responses in subjects. Following an initial vaccination, subjects may receive a boost in about 4 weeks.

The amount of antigen in each vaccine dose is an amount which induces an immunoprotective or therapeutically effective response without significant adverse side effects in typical female vaccinees.

Generally it is expected that each dose will comprise 1-1000µg of antigen, preferably 2-100µg, most preferably 4-40µg. The TH-1 inducing adjuvant, for example 3-DMPL, will normally be present in a range of 10-200µg, preferably 25-75µg, especially about 50µg per dose.

The amount of carrier may vary and may be selected according to the knowledge of one skilled in the art. If aluminium hydroxide (alum) or aluminium phosphate is used the amount employed will generally be in the range 100-1000µg, for example 250 - 750µg, preferably about 500µg per vaccine dose.

Typical amounts of each component in the vaccine are antigen (20µg), alum (500µg) and an adjuvant, especially a TH-1 inducing adjuvant such as 3-DMPL (50µg).

In one preferred aspect the vaccine for use in the invention comprises gD2t, 3-DMPL (especially small particle 3-DMPL) and aluminium hydroxide (alum).

In one preferred regimen the vaccine may be given at intervals of 0, 1 and 6 months. Other dosing regimens, including booster doses, may also be used. The vaccine may be administered intramuscularly.

The manufacture of a vaccine according to the invention may be accomplished by conventional techniques, such as described in WO 92/16231. The method typically involves mixing one or more antigens derived from or associated with an STD with an adjuvant, especially a TH-1 inducing adjuvant, and optionally a carrier as hereinabove described. The resulting vaccine composition may be used for administration to female subjects according to the method of the invention, especially sexually active women suffering from or at risk of contracting an STD.

Generally the women will be in an age range of 12-70 years, more usually adolescents and women of 60 or less, for example 14-60, typically 18-45 as in the study described below. In one aspect a suitable group of women includes those suffering from or at risk of contracting genital herpes infection. The use of the invention may, for example, be applied in seronegative healthy consorts of subjects with genital herpes disease.

The invention is illustrated, without limitation, by the following examples, showing results when a herpes vaccine was administered to female subjects. Similar results may be obtained with vaccines against other STDs such as HPV and with combination or polyvalent vaccines against more than one STD, especially combination vaccines comprising HSV-2 gD or immunological fragments thereof such as gD2t as hereinabove described.

### EXAMPLE 1 - DESIGN OF STUDY

- **Vaccine under study**: SmithKline Beecham Biologicals Herpes simplex candidate vaccine (gD2t-20µg) with Alum (500µg) and 3-DMPL (50µg).
- **Title**: A double-blind, randomized, placebo-controlled study to evaluate the efficacy of SmithKline Beecham Biologicals' Herpes Simplex candidate vaccine (gD2t) with 3-DMPL to prevent genital herpes disease in healthy consorts of subjects with genital herpes disease.
- **Indication/study population**: Healthy adult volunteers, male and female, aged 18 to 45 years with negative serological markers of Herpes Simplex infection (HSV-1 and -2) and whose consort has clinical genital herpes disease.

### Objectives of the study

### Primary

To compare with placebo, during the 17 month period starting one month after the second vaccination, the protective efficacy of gD-Alum-3-DMPL vaccine to prevent genital herpes clinical disease.

### Secondary

To compare with placebo, starting one month after the second vaccination, the protective efficacy of gD-Alum-3-DMPL vaccine to prevent genital herpes infection.

To compare with placebo after the full vaccination course, the protective efficacy of gD-Alum-3-DMPL vaccine to prevent genital herpes infection.

To compare with placebo, after the full vaccination course, the protective efficacy of gD-Alum-3-DMPL vaccine to prevent genital herpes infection during a period of extended clinical follow-up.

To compare with placebo, after the full vaccination course, the protective efficacy of gD-Alum-3-DMPL vaccine to prevent genital herpes disease.

To compare with placebo, after the full vaccination course, the protective efficacy of gD-Alum-3-DMPL vaccine to prevent genital herpes clinical disease during a period of extended clinical follow-up.

To evaluate, starting one month after the second vaccination, the time to occurrence of disease in each group.

To evaluate, starting one month after the second vaccination, the time to occurrence of infection in each group.

To evaluate, in each group, the number of typical and atypical cases of genital herpes disease.

To evaluate the severity of primary disease in both groups.

To evaluate the humoral and cellular immune response (excluding subjects from study centers initiated after July 1st, 1995) of the vaccine.

To determine serological or immunological correlates for protective efficacy (excluding subjects from study centers initiated after July lst, 1995).

In case of primary disease or infection, to evaluate the number of subsequent recurrences in the two groups.

To evaluate the safety and reactogenicity of SmithKline Beecham Biologicals' herpes simplex candidate vaccine (with 3-DMPL) in healthy HSV seronegative subjects.

To evaluate the number of cases of oro-labial (or non-genital) herpes disease.
To compare with placebo, starting one month after the second vaccination, the protective efficacy of gD-Alum-3-DMPL to prevent suspected genital herpes signs and symptoms associated with either Western Blot seroconversion to non-vaccinal antigens or with the detection of HSV DNA in a genital swab by PCR.
To evaluate the incidence of genital herpes disease and HSV infection in vaccine recipients during the period of extended clinical follow-up.

### Study design

Double-blind, randomized, placebo-controlled study.
Vaccination schedule : 0-1-6 months.
Initial follow-up period - 17 months for each subject starting 1 month after the second vaccination.
Extended follow-up period - 24 months for each subject (from the month 19 visit to the month 43 visit)

Phase A (double blind, vaccine and placebo recipients) - ends when the last subject enrolled completes the initial follow-up period (around the time that the study is unblinded for analysis).

Phase B (open, vaccine recipients only) - begins when the last subject enrolled completes the initial follow-up period (month 19 visit) and ends when the last subject enrolled completes the month 43 visit.

Because there may be a period of several months between the date that the last subject enrolled completes the initial follow-up period and the date that the study is fully unblinded for analysis, (due to the time required for encoding and cleaning of all of the study data collated during the initial follow-up period and phase A of the extended follow-up period), the initial part of phase B of the extended follow-up period may include both vaccine and placebo recipients.
- 2 groups :: 1. gD2t-Alum-3-DMPL
Alum-3-DMPL as placebo

Schematic of HSV-007 Study Design - Study periods: Vaccination (V) phase; Initial follow-up* (initial f/u*); Extended follow-up phase A; Extended follow-up phase B.
*Note: The modified "initial follow-up period" now includes months 2-19
- Number of subjects: 800 couples will be enrolled into the study to allow for at least 640 evaluable subjects.
- Primary efficacy endpoint: During the 17-month period, starting one month after the second vaccination (months 2-19), the primary efficacy end-point will be as follows:

### Prevention of disease:

A comparison between the two groups of the number of subjects with at least one compatible symptom of genital herpes disease AND either a concurrent positive culture OR appearance of antibodies to non-vaccinal antigens by Western Blot within six months and positive local detection of herpes simplex DNA by Polymerase Chain Reaction (PCR).

| | Clinical Symptom | Culture | Antibodies to non-vaccinal antigens | PCR |
|---|---|---|---|---|
| Disease | + | + | +/- | NA |
| | + | - | + | + |

| | | | | |
|---|---|---|---|---|
| NA : Not Applicable | | | | |

### Secondary efficacy endpoints

### 1) Prevention of infection:

A comparison will be made (between vaccine and placebo groups), of the number of subjects who develop antibodies to non-vaccinal antigens (seroconversion) and of subjects who develop disease (culture proven).

This endpoint will be evaluated for the following periods:
Initial period of follow-up (months 2-19)
Months 7-19
Phase A of the extended follow-up
Initial period of follow-up (months 2-19) and phase A of the extended
follow-up combined.
Months 7-19 and phase A of the extended follow-up combined.

The analysis of data from phase A of the extended follow-up period will include all events occurring after each subject's month 19 visit and the end of phase A (when the last subject enrolled completes the month 19 visit).

**Case definition**

| | | Clinical Symptom | Culture | Antibodies to non-vaccinal antigens | PCR |
|---|---|---|---|---|---|
| Infection | Disease | + | + | +/- | NA |
| Infection | Disease | + | - | + | + |
| Infection | | +/- | NA | + | NA |

| | | | | | |
|---|---|---|---|---|---|
| NA : Not Applicable | | | | | |

### 2) Prevention of disease between months 7-19

A comparison with placebo after the full vaccination course (months 7-19) of the number of subjects with at least one compatible symptom of genital herpes disease AND either a concurrent positive culture OR appearance of antibodies to non-vaccinal antigens by Western Blot and positive local detection of herpes simplex DNA by Polymerase Chain Reaction (PCR).

### 3) Prevention of disease during phase A of the extended follow-up period

During phase A of the extended follow-up period, a comparison will be performed between the two groups of the number of subjects with at least one compatible symptom of genital herpes disease AND either a concurrent positive culture OR appearance of antibodies to non-vaccinal antigens by Western Blot and positive local detection of herpes simplex DNA by Polymerase Chain Reaction (PCR).

In addition, this endpoint will also be evaluated for the new initial follow-up period (months 2-19) and phase A of the extended follow-up period combined and also for months 7-19 and phase A of the extended follow-up combined.
4) To evaluate during the 17 month period starting one month after the second vaccination, in each group, the time to occurrence of genital herpes disease.
5) To evaluate during the 17 month period starting one month after the second vaccination, in each group, the time to occurrence of genital herpes infection.
6) To evaluate in each group the number of cases of typical genital herpes clinical disease and of atypical genital herpes disease.
   The case definitions are described in the primary end-point.
7) To evaluate in each group the patient's subjective local and general signs and symptoms of genital HSV disease and their duration.
8) To evaluate the humoral (anti-gD2 antibodies by ELISA and anti-HSV neutralizing antibodies) and cellular (lymphoproliferation, secretion of gamma interferon) response to the vaccine (excluding subjects from study centers initiated after July 1, 1995).
9) If clinical efficacy is demonstrated, serological and immunological markers will be extensively evaluated using the sera and Peripheral Blood Lymphocytes stored when scheduled, in an attempt to determine correlates between protective efficacy and laboratory parameters (excluding subjects from study centers initiated after July 1, 1995).
10) In case of primary disease or infection, to evaluate the number of subsequent recurrences of genital herpes in each group.
11) The local and general reactogenicity and the safety will be evaluated after each vaccination by recording the local and general signs and symptoms after each dose and the adverse experiences during the study course. The haematological and biochemical parameters will be checked at baseline and after the last vaccination.
   During the extended follow-up period, all serious adverse experiences reported by vaccine recipients will be recorded.
12) To evaluate the number of clinical cases of non-genital herpes disease, including oro-labial herpes disease, in each group.
13) To compare with placebo, during the 17 month follow-up period, starting one month after the second vaccination, the number of vaccine recipients who develop genital herpes signs and symptoms associated with either seroconversion to non-vaccinal antigens by Western Blot (within a six month period from the onset of genital herpes signs or symptoms) or with the detection of HSV DNA in a genital swab by PCR.
14) During phase B of the extended follow-up period, the number of vaccine recipients who develop antibodies to non-vaccinal antigens (seroconversion) and of subjects who develop disease (culture proven) will be analyzed in relationship to the interval since administration of last vaccination. These data will be used to calculate the attack rate of genital herpes disease and infection.

### EXAMPLE 2

The analysis of the primary endpoint is based on comparison of attack rates between the vaccine and placebo groups as described in the RAP. The analysis of the secondary endpoints is based on either comparison of attack rates or comparison of time to occurrence of disease or infection endpoints as described below.
Statistical tests are two-sided and performed using SAS software and an α-level of 0.05. It should be noted that many statistical analyses are reported, but for the secondary endpoints the error rate (α) is not under control. Since no adjustments of the α were performed for the secondary endpoints, the p-values must be interpreted cautiously and as descriptive only.

### Populations analysed for Vaccine Efficacy

Efficacy analyses are performed on two subject populations: the intention-to-treat population (ITT) and the according-to-protocol population (ATP). The ATP group is also referred to hereinbelow as the per-protocol (PP) group.

The analysis of the according-to-protocol population is the primary analysis. The definition of the ATP (or PP) population is defined by the study period under consideration:
1) For the period between months 2-19, the ATP population consists of subjects:
   - who meet all protocol eligibility criteria
   - who have received three doses of vaccine/placebo
   - or who have received two doses of vaccine/placebo and for whom the considered event (disease or infection) has occurred prior to the month 6 visit
   - for whom the considered event (disease or infection) has not occurred before the start of the month 2-19 period.
2) For the period between months 7-19, the ATP population consists of subjects:
   - who meet all protocol eligibility criteria
   - who have received three doses of vaccine/placebo
   - for whom the considered event (disease or infection) has not occurred before the start of the month 7 - 19 period.

The analysis of the ITT population is considered as the secondary analysis. This analysis includes all subjects who received at least one dose of study vaccine and have at least one on-vaccine assessment.

The purpose of the two analyses is to ensure that protocol violations, subject dropouts and withdrawals are not treatment related and do not lead to any selection bias in the efficacy results.

The populations to be included in the immunogenicity and safety analyses will be fully described in the final study report.

### Evaluation periods

The evaluation periods during which analyses are performed include:
- months 2-19 (ATP population)
- months 7-19 (ATP population)
- months 0 - 19 (ITT population)

Selected results are shown below, together with a summary of the overall conclusions of the study.

**Table 1a. Adjustment of vaccine effect on the occurrence of genital herpes disease by gender- ITT population**

| **Terms fitted in the model** | **Deviance** | **Degrees of freedom** | **p-value** |
|---|---|---|---|
| treatment group | 320.5561 | 845 | |
| treatment group, gender | 317.2083 | 844 | 0.067 |
| treatment group, gender, and the interaction | 312.0443 | 843 | 0.023 |

**Table 1b. Adjustment of vaccine effect on the occurrence of genital herpes infection by gender- ITT population**

| **Terms fitted in the model** | **Deviance** | **Degrees of freedom** | **p-value** |
|---|---|---|---|
| treatment group | 510.8654 | 845 | |
| treatment group, gender | 494.9266 | 844 | < 0.00 1 |
| treatment group, gender, and the interaction | 491.5551 | 843 | 0.066 |

### Distribution of Genital Herpes Disease and HSV Infection Cases by Treatment Group

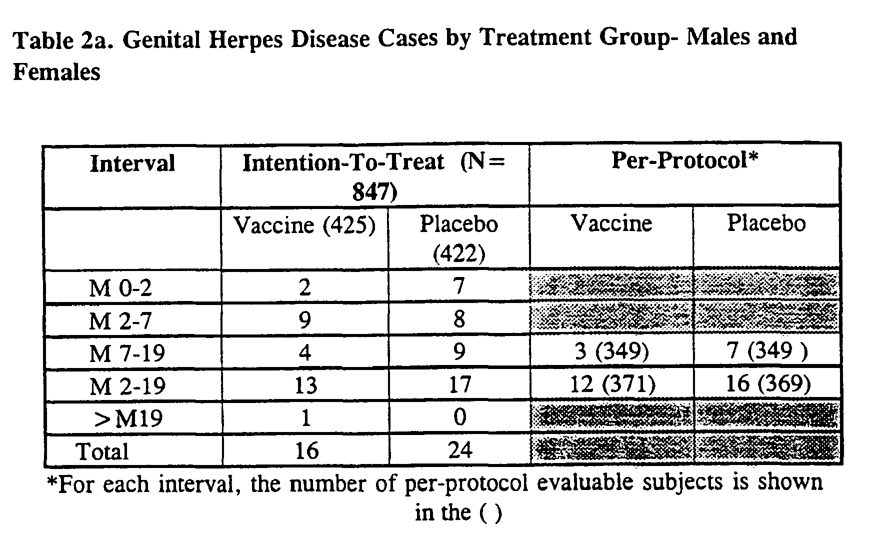

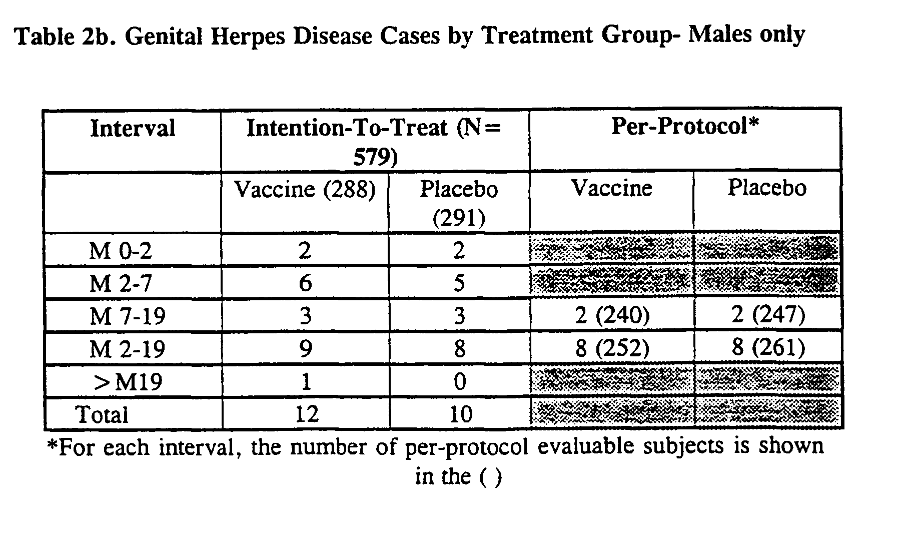

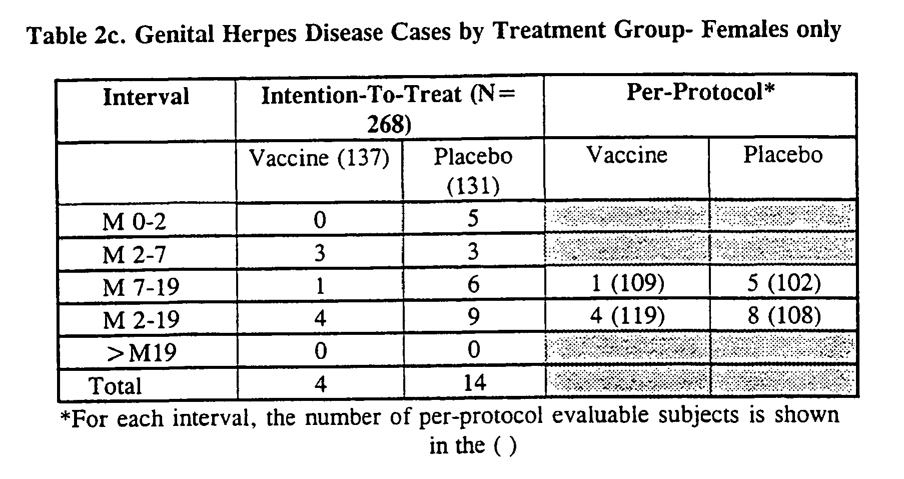

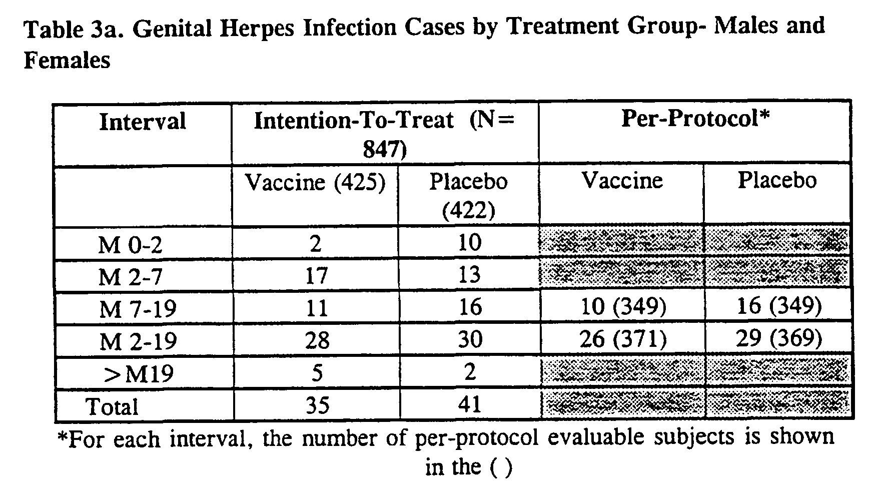

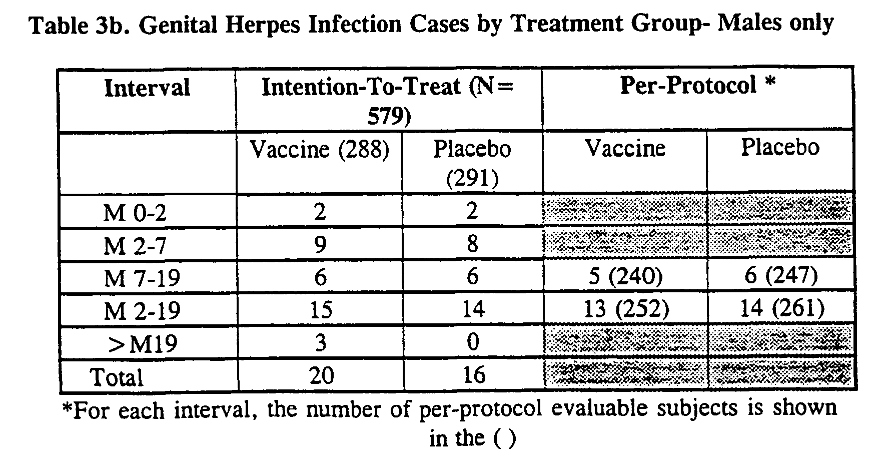

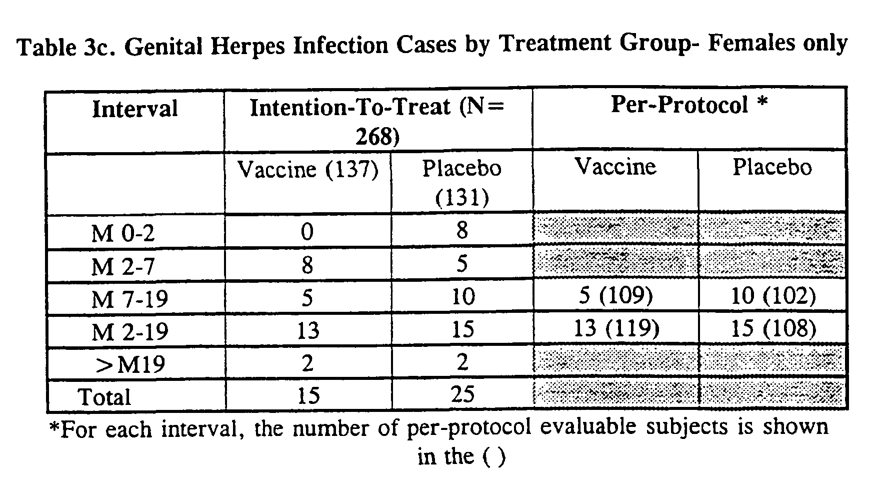

### Preliminary Efficacy Analysis

### Primary efficacy endpoint

During the 17-month period, starting one month after the second vaccination (months 2-19), the primary efficacy end-point will be as follows:

### Prevention of disease:

A comparison between the two groups of the number of subjects with at least one compatible symptom of genital herpes disease AND either a concurrent positive culture OR appearance of antibodies to non-vaccinal antigens by Western Blot within six months and positive local detection of herpes simplex DNA by Polymerase Chain Reaction (PCR).

**Table 4a. Prevention of Genital Herpes Disease- Males and Females**

| **Interval** | **Population** | **Efficacy (95% CI)** |
|---|---|---|
| All | ITT | 33.8 (-22.8, 64.3 %) |
| M 2-19 | PP | 25.4% (-55.5, 64.2%) |

**Table 4b. Prevention of Genital Herpes Disease- Males only**

| **Interval** | **Population** | **Efficacy (95% CI)** |
|---|---|---|
| All | ITT | -21.2 (-176.2, 46.8%) |
| M 2-19 | PP | 3.6% (-171.7, 60.5%) |

**Table 4c. Prevention of Genital Herpes Disease- Females only**

| **Interval** | **Population** | **Efficacy (95% CI)** |
|---|---|---|
| All | ITT | 72.7% (19.1, 90.8%) |
| M 2-19 | PP | 54.6 % (-46.4, 85.9%) |

### Secondary efficacy endpoints

### 1) Prevention of infection:

A comparison will be made (between vaccine and placebo groups), of the number of subjects who develop antibodies to non-vaccinal antigens (seroconversion) and of subjects who develop disease (culture proven) for the initial period of follow-up (months 2-19).

**Table 5a. Prevention of Genital Herpes Infection- Males and Females**

| **Interval** | **Population** | **Efficacy (95% CI)** |
|---|---|---|
| All | ITT | 15.2 % (-30.4, 44.0%) |
| M 2-19 | PP | 10.8 % (-48.4, 46.4%) |

**Table 5b. Prevention of Genital Herpes Infection- Males only**

| **Interval** | **Population** | **Efficacy (95% CI)** |
|---|---|---|
| All | ITT | -26.3% (-138.8. 33.2%) |
| M 2-19 | PP | 3.8% (-100.5, 53.9%) |

**Table 5c. Prevention of Genital Herpes Infection- Females only**

| **Interval** | **Population** | **Efficacy (95% CI)** |
|---|---|---|
| All | ITT | 42.6% (-3.9, 68.3%) |
| M 2-19 | PP | 21.3% (-57.7. 60.8%) |

### 2) Prevention of infection:

A comparison will be made (between vaccine and placebo groups), of the number of subjects who develop antibodies to non-vaccinal antigens (seroconversion) and of subjects who develop disease (culture proven) for months 7-19.

**Table 6a. Prevention of Genital Herpes Infection- Males and Females**

| **Interval** | **Population** | **Efficacy (95% CI)** |
|---|---|---|
| M 7-19 | PP | 37.5 % (-35.8, 71.2%) |

**Table 6b. Prevention of Genital Herpes Infection- Males only**

| **Interval** | **Population** | **Efficacy (95% CI)** |
|---|---|---|
| M 7-19 | PP | 14.2% (-177.3, 73.5%) |

**Table 6c. Prevention of Genital Herpes Infection- Females only**

| **Interval** | **Population** | **Efficacy (95% CI)** |
|---|---|---|
| M 7-19 | PP | 53.2% (-32.2, 83.4%) |

### 3) Prevention of disease between months 7-19

A comparison with placebo after the full vaccination course (months 7-19) of the number of subjects with at least one compatible symptom of genital herpes disease AND either a concurrent positive culture OR appearance of antibodies to non-vaccinal antigens by Western Blot and positive local detection of herpes simplex DNA by Polymerase Chain Reaction (PCR).

**Table 7a. Prevention of Genital Herpes Disease- Males and Females**

| **Interval** | **Population** | **Efficacy (95% CI)** |
|---|---|---|
| M 7-19 | PP | 57.1 % (-64.4, 88.8 %) |

**Table 7b. Prevention of Genital Herpes Disease- Males only**

| **Interval** | **Population** | **Efficacy (95% CI)** |
|---|---|---|
| M 7-19 | PP | -2.9% (-624.7, 85.4%) |

**Table 7c. Prevention of Genital Herpes Disease- Females only**

| **Interval** | **Population** | **Efficacy (95% CI)** |
|---|---|---|
| M 7-19 | PP | 81.3% (-57.5, 97.8%) |

To evaluate during the 17 month period starting one month after the second vaccination, in each group, the time to occurrence of genital herpes disease.

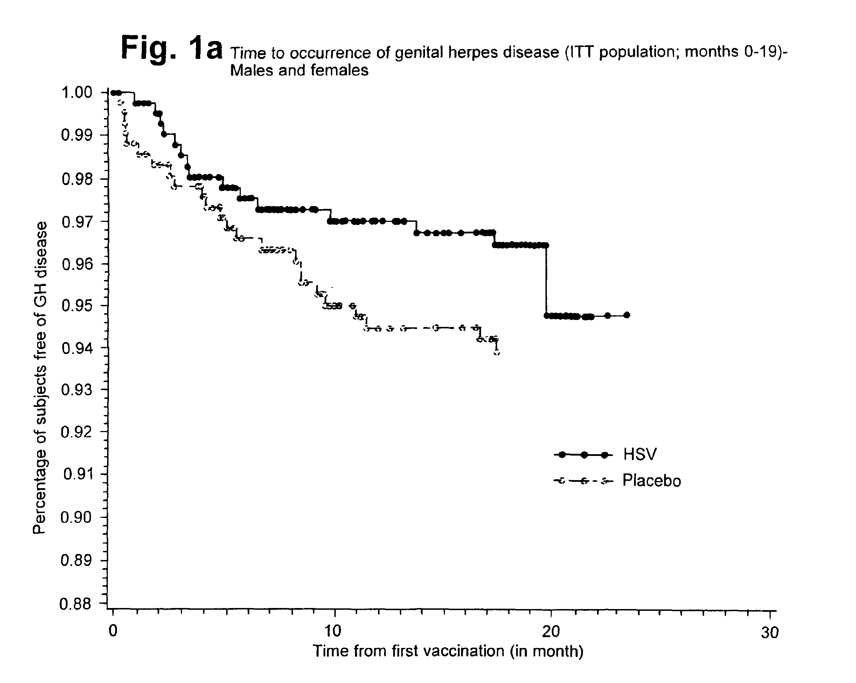

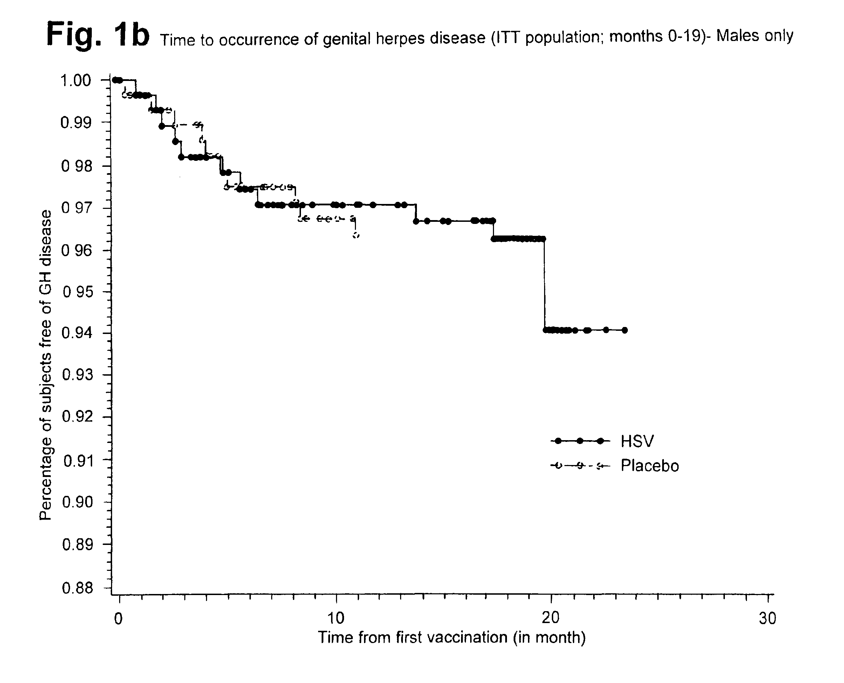

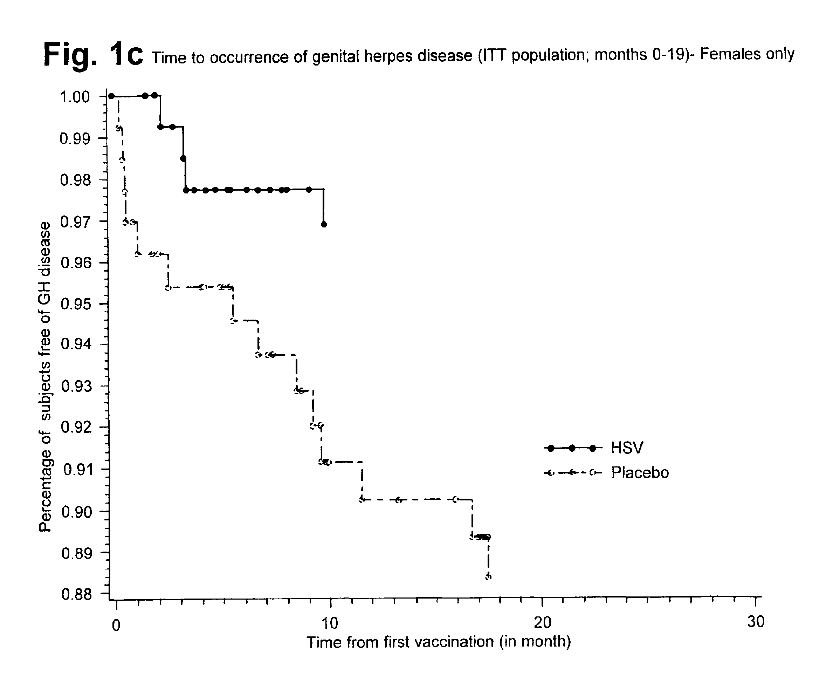

Time to occurrence of genital herpes disease for the ITT population is shown in figures 1a (males and females), 1b (males only), and 1c (females only). The efficacy analyses of time to occurrence of genital herpes disease for the ITT population is shown in tables 8a (males and females), 8b (males only), and 8c (females only). Time to occurrence was not analyzed for the per-protocol populations (months 2-19) since an early difference in survival without disease was observed between vaccine and placebo recipients prior to month 2.
Note: the time to occurrence analysis excludes genital herpes disease cases occurring after month 19.

**Table 8a. Prevention of Genital Herpes Disease by Time to Occurrence-Males and Females**

| **Interval** | **Population** | **p-value (Log Rank test)** | **Efficacy (95% CI)** |
|---|---|---|---|
| M 0-19 | ITT | 0.1432 | 37.96% (-18.27. 67.45%) |

**Table 8b. Prevention of Genital Herpes Disease by Time to Occurrence-Males only**

| **Interval** | **Population** | **p-value (Log Rank test)** | **Efficacy (95% CI)** |
|---|---|---|---|
| M 0-19 | ITT | 0.8025 | -11.54 % (-162.55, 52.63%) |

**Table 8c. Prevention of Genital Herpes Disease by Time to Occurrence-Females only**

| **Interval** | **Population** | **p-value (Log Rank test)** | **Efficacy (95% CI)** |
|---|---|---|---|
| M 0-19 | ITT | 0.013 | 73.24% (18.69, 91.19%) |

### Summary and conclusions after detailed analysis of the results of the trial

### Demographic characteristics and risk factors evaluation

Overall, of the 847 (425 vaccine and 422 placebo) subjects enrolled, 697 (344 vaccine and 353 placebo) subjects completed the study through to month 19. One hundred and fifty (150) subjects dropped out of the study; none of the drop outs resulted from a serious adverse event.

Three hundred and seventy (370) subjects in the vaccine group and 369 in the placebo group were evaluable in the month 2 - 19 ATP population. Treatment groups were balanced for all demographic characteristics and the selection of protocol compliers for the ATP population analysis did not result in the introduction of bias by treatment groups.

Risk factors that might impact on the rate of acquisition of genital herpes disease or infection were assessed and included duration of relationship prior to study entry, the mean time until separation from the source partner, the frequency of sexual intercourse (at baseline and during the efficacy follow-up period), and the frequency of condom use (at baseline and during the efficacy follow-up period).

These results indicate that the vaccine and placebo groups were balanced at baseline for all risk factors and the balance was maintained during the study. The similarity of the ITT population profile to that of the ATP population in terms of risk factors also confirms that the elimination of the non-compliers to the protocol did not bias the treatment groups.

Sub-analyses by gender indicates that within each gender group, risk factors that might impact on the acquisition of genital herpes disease or infection are balanced by treatment group.

### Primary efficacy endpoint analysis

The analysis of the primary efficacy endpoint does not demonstrate vaccine efficacy against genital herpes in a combined population of male and female seronegative healthy consorts of subjects with genital herpes disease.

The results of the primary efficacy endpoint analysis are summarised as follows:
1) The relative vaccine efficacy in the overall population (month 2 - 19 ATP) is 25.4 % (95 % CI: -55.5, 64.2; *p* = *0. 449).* The relative vaccine efficacy for the ITT population is 37.9% (95% CI: -16.6, 67.0; *p* = *0.143).*
2) A statistically significant gender by group interaction on the efficacy analysis of the ITT population (p=0.03).
3) A separate analysis by gender shows a vaccine efficacy of 54.2 % in the month 2-19 ATP female population (95 % CI: -47.7, 85.8; *p* = *0. 238)* and a statistically significant vaccine efficacy of 72.7% (95 % CI: 19.1, 90.8; *p = 0.014*) in the female ITT population. In the male population, there is no evidence of vaccine efficacy. In the month 2-19 ATP male population vaccine efficacy is 3.6% (95% CI: -171, 60.5) and -11.1%, (95% CI; -157.6, 52.1) in the ITT male population.

Several baseline covariates were investigated to determine whether they might influence efficacy outcomes: gender, age, frequency of condom use at baseline, frequency of sexual intercourse and duration of relationships prior to study entry. Tendency towards vaccine efficacy was associated with the female gender, age above 30 years, infrequent condom use, sexual intercourse of less than the median frequency and shorter duration of relationships. These observations applied to both ATP and ITT populations.

### Secondary efficacy endpoint analyses

### Prevention of genital herpes disease (month 7 - 19)

After 3 doses of the vaccine (between study months 7 - 19), vaccine efficacy of 81.1 % (95 % CI: -58.9, 97.8) was observed for prevention of genital herpes disease in females (p = 0.111). No tendency toward efficacy was observed in males during the month 7 - 19 observation period (-2.9% vaccine efficacy, 95 % CI: -624.7, 85.4; *p* = *0.99*). This tendency towards vaccine efficacy in the month 7 - 19 female ATP population is consistent with the observation of vaccine efficacy in females in the ITT analysis of the primary endpoint.

### Prevention of HSV infection

A comparison was made between vaccine and placebo groups for the efficacy of the vaccine in preventing HSV infection. Overall, there was no vaccine efficacy against HSV infection. However, consistent with the disease endpoint analyses, a tendency toward vaccine efficacy against HSV infection was suggested in females in the ITT population (vaccine efficacy of 46.0 % , 95 % CI: -2.1, 71.4; *p = 0.072)* and in the month 7 - 19 ATP population (vaccine efficacy of 52.8%, 95% CI: -33.4, 83.3; *p* = *0.184*).

### Time to occurrence of genital herpes

Time to occurrence of genital herpes disease was calculated from study entry until the occurrence of the disease. The main analysis has been performed by the logrank test; Kaplan-Meier curves were plotted for each group. In females (month 2 - 19 ATP population), separation of the curves denoting the occurrence of disease cases is apparent from approximately nine months with disease cases continuing to occur in the placebo group. Vaccine efficacy is estimated at 53.6% (95% CI: -54.2, 86.0) in females. In the ITT female population where the separation of the vaccine and placebo curves is apparent from month 0, a statistically significant vaccine efficacy is estimated at 73.2% (95% CI: 18.7, 91.2; p=0.013). No vaccine efficacy is observed for the male population. Again, the results of the "time to occurrence" analysis is consistent with the primary endpoint analysis.

### Severity of Genital herpes disease

Parameters including, duration of lesions, duration of symptoms per episode, number of symptoms per episode and intensity of symptom per episode were used to assess severity of disease in both treatment groups. In the combined month 2 - 19 ATP population, duration of symptoms per episode is significantly longer in the small number of cases occurring in the vaccine group (p = 0.031). The gender specific severity data also reveals that in females, there is statistically significant higher number of genital herpes disease lesions per episode (*p* = *0.010*) in the vaccine group. These observations suggests that while vaccination may prevent mild to moderate disease in the vaccine group, disease with more severe manifestations were not prevented by vaccination.

### Overall Conclusions

The analysis demonstrates that while there may be a tendency toward vaccine efficacy against genital herpes, the primary endpoint analysis does not demonstrate vaccine efficacy in a combined population of male and female seronegative healthy consorts of subjects with genital herpes disease. However, a separate sub-analysis by gender group, based on observed gender interaction, **surprisingly shows a tendency towards vaccine efficacy in females** which is statistically significant in the ITT population. There is no evidence of vaccine efficacy in the male population.

### ADDITIONAL REFERENCES

1. Washington AE, Johnson RE and Sanders LL. Chlamydia trachomatis infections in the United States: what are they costing us. JAMA 1987, 257, 2070-2072.
2. Grayston JT and Wang SP. New knowledge of Chlamydiae and the diseases they cause. The Journal of Infectious Diseases 1975, 132: 87-105.
3. Grayston JT, Wang SP, Yeh LJ, and Kuo CC. Importance of reinfection in the pathogenesis of trachoma. Reviews of Infectious Diseases 1985, 7, 717-725
4. Morrison RP, RJ Belland, K Lyng and HD Caldwell. Chalmydial disease pathogenesis. The 57-kD Chlamydial hypersensitivity antigen is a stress response protein. J. Exp. Med. 1989, 170, 1271-1283
5. Blander SJ and Amortegui AJ. Mice immunized with a chlamydial extract have no increase in early protective immunity despite increased inflammation following genital infection by the mouse pneumonitis agent of Chlamydia trachomatis. Infec. Immun. 1994, 62, 3617-3624. µg
6. Wang SP, Kuo CC, Barnes RC, Stephens RS and Grayston JT. Immunotyping of Chlamydia trachomatis with monoclonal antibodies. The Journal of Infectious Diseases 1985, 152, 791-800.
7. Bavoil P, Ohlin A, and Schachter J. Role of disulfide bonding in outer membrane structure and permeability in Chlamydia trachomatis. Infect. Immun. 1984, 44, 479-485.
8. Hatch TP, Miceli M, Sublett JE. Synthesis of disulfide-bonded outer membrane proteins during development cycle of Chlamydia psittaci and Chlamydia trachomatis. J. Bacteriol. 1986, 165, 379-385.
9. Stephens RS, R Sanchez-Pescador, EA Wagar, C Inouye and MS Urdea. Diversity of Chlamydia trachomatis Major Outer Membrane Protein genes. J. Bacteriol. 1987, 169, 3879-3885.
10. Yuan Y, Zhang YX, Watkins NG, and Caldwell HD. Nucleotide and deduced amino acid sequences for the four variable domains of the major outer mebrane proteins of the 15 Chlamydia trachomatis serovars. Infect. Immun. 1989, 57, 1040-1049.
11. Baehr W, Zhang YX, Joseph T, Su H, Nano FE, Everett EK and Calwell HD. Mapping antigenic domains expressed by Chlamydia trachomatis major outer membrane protein genes. Proc. Natl Acad. Sci. USA 1988, 85, 4000-4004
12. Lucero ME and Kuo CC. Neutralization of Chlamydia trachomatis cell culture infection by serovar-specific monoclonal antibodies. Infect. Immun. 1985, 50, 595-597
13. Zhang YX, Stewart S, Joseph T, Taylor HR and HD Caldwell. Protective monoclonal antibodies recognize epitopes located on the major outer membrane protein of Chlamydia trachomatis. J. Imunol. 1987, 138, 575-581.
14. Peterson E, Zhong G, Carlson E and de la Maza LM. Protective role of magnesium in the neutralization by monoclonal antibodies of Chlamydia trachomatis infectivity. Infect. Immun. 1988, 56, 885-891.
15. Zhang YX, Stewart SJ and Caldwell HD. Protective monoclonal antibodies to Chlamydia trachomatis serovar- and serogroup- specific major outer membrane protein determinants. Infect. Immun. 1989, 57, 636-638
16. Allen JE, RM Loksley and RS Stephens. A single peptide from the major outer membrane protein of Chlamydia trachomatis elicits T cell help for the production of antibodies to protective determinants. J. Immunol. 1991, 147, 674-679
17. Su H, RP Morrison, NG Watkins and HD Caldwell. Identification and characterization of T helper cell epitopes of the major outer membrane protein of Chlamydia trachomatis. J. Exp. Med. 1990, 172, 203-212
18. Manning DS and SJ Stewart. Expression of the major outer membrane protein of Chalmydia trachomatis in Escherichia coli. Infect. Immun. 1993, 61, 4093-4098.
19. Koehler JE, Birkelund S and Stephens RS. Overexpression and surface localization of the Chlamydia trachomatis major outer membrane protein in Escherichia coli. Molecular Microbiology 1992, 6, 1087-1094
20. Pickett MA, ME Ward and IN Clarke. High level expression and epitope localization of the major outer membrane protein of Chlamydia trachomatis serovar L1. Molecular Microbiology 1988, 2, 681-685.
21. Taylor HR, J Whittum-Hudson, J Schachter, HD Caldwell and RA Prendergast. Oral immunization with chlamydial major outer membrane protein (MOMP). Investigative Ophthalmology and visual Science 1988, 29, 1847-1853
22. Batteiger BE, RG Rank, PM Bavoil and LSF Soderberg. Partial protection against genital reinfection by immunization of guinea-pig with isolated mouter membrane proteins of the chlamydial agent of guinea-pig inclusion conjunctivitis. Journal of General Microbiology 1993, 139, 2965-2972.
23. Tuffrey M, F Alexander, W Conlan, C Woods and M Ward. Heterotypic protection of mice against chlamydial salpingitis and colonization of the lower genital tract with a human serovar F isolate of Chlamydia trachomatis by prior immunization with recombinant L1 major outer-membrane protein. Journal of General Microbiology 1992, 138, 1707-1715.
24. Tuffrey M, P Falder, J Gale and D Taylor -Robinson. Salpingitis in mice induced by human strains of Chlamydia trachomatis. Br. J. exp. Path. 1986, 67, 60S-616.
25. Tuffrey M, P Falder, J Gale, R Quinn and D Taylor -Robinson.Infertility in mice infected genitally with a human strain of Chlamydia trachomatis . Br. J. exp. Path. 1986, 78, 251-260
26. Ramsey KH, LSF Soderberg and RG Rank. Resolution of Chlamydial genital infection in B-cell deficient mice and immunity to reinfection. Infect. Immun. 1988, 56, 1320-1325.
27. Rank RG, LSF Soderberg and AL Barron. Chronic chlamydial genital infection in congenitally athymic nude mice. Infect. Immun. 1985, 48, 847-849
28. Igietseme JU and RG Rank. Susceptibility to reinfection after a primary chlamydial genital infection is associated with a decrease of antigen-specific T cell in the genital tract. Infect. Immun. 1991, 59, 1346-1351
29. Igietseme JU, KH Ramsey, DM Magee, DM Williams TJ Kincy and RG Rank. Resolution of murine chlamydial genital infection by the adoptive transfer of a biovar-specific, TH1 Lymphocyte clone. Regional Immunology 1993, 5, 317-324.
30. Igietseme JU, DM Magee, DM Williams and RG Rank. Role for CD8+ T cell in antichlamydial immunity defined by chlamydial-specific T-lymphocyte clones. Infect. Immun. 1994, 62 , 5195-5197.

## Claims

1. Use of an HSV glycoprotein D or an immunological fragment thereof and an adjuvant in the preparation of a vaccine for administration to an HSV1-/2- human female subject for the prevention of genital herpes disease.

2. Use according to claim 1 in which the adjuvant is a TH-1 inducing adjuvant.

3. Use according to claim 1 or 2 in which the HSV glycoprotein D is a truncated glycoprotein.

4. Use according to claim 3 in which the truncated glycoprotein is HSV gD2 and is devoid of the C-terminal anchor region (gD2t).

5. Use according to any preceding claim in which the vaccine further includes an antigen derived from HPV.

6. Use according to any preceding claim wherein the antigen or combination of antigens is formulated with a suitable carrier.

7. Use according to claim 6 wherein the carrier is aluminium hydroxide (alum), aluminium phosphate or an oil in water emulsion.

8. Use according to any preceding claim wherein the adjuvant is the TH-1 inducing adjuvant 3D-MPL.

9. Use according to claim 8 in which the particles of 3D-MPL are small enough to be sterile filtered through a 0.22 micron membrane.

10. Use according to any preceding claim in which the vaccine formulation comprises gD2t (1-1000µg), 3D-MPL (10-200µg) and an aluminum salt (100-1000µg).

11. Use according to claim 10 in which the vaccine formulation comprises gD2t (20µg), 3D-MPL (50µg) and alum (500µg).

12. Use according to any preceding claim wherein the vaccine formulation is administered to, or manufactured for administration to, female subjects at intervals of 0, 1 and 6 months.

13. Use according to any preceding claim wherein the vaccine formulation is administered intramuscularly.

## Patentansprüche

1. Verwendung eines HSV-Glycoproteins D oder eines immunologischen Fragments davon und eines Adjuvans für die Herstellung eines Vakzins zur Verabreichung an ein HSV1-/2-menschliches weibliches Subjekt zur Prävention einer genitalen Herpeserkrankung.

2. Verwendung gemäß Anspruch 1, worin das Adjuvans ein TH-1 induzierendes Adjuvans ist.

3. Verwendung gemäß Anspruch 1 oder 2, worin das HSV-Glycoprotein D ein trunkiertes Glycoprotein ist.

4. Verwendung gemäß Anspruch 3, worin das trunkierte Glycoprotein HSV gD2 ist, dem die C-terminale Ankerregion (gD2t) fehlt.

5. Verwendung gemäß einem der vorstehenden Ansprüche, worin das Vakzin weiterhin ein Antigen beinhaltet, das von HPV abstammt .

6. Verwendung gemäß einem der vorstehenden Ansprüche, wobei das Antigen oder die Kombination der Antigene mit einem geeigneten Träger formuliert ist.

7. Verwendung gemäß Anspruch 6, wobei der Träger Aluminiumhydroxid (Alaun), Aluminiumphosphat oder eine Öl-in-Wasser-Emulsion ist.

8. Verwendung gemäß einem der vorstehenden Ansprüche, worin das Adjuvans das TH-1 induzierende Adjuvans 3D-MPL ist.

9. Verwendung gemäß Anspruch 8, worin die Teilchen von 3D-MPL klein genug sind, um durch eine 0,22 µm Membran steril filtriert zu werden.

10. Verwendung gemäß einem der vorstehenden Ansprüche, worin die Vakzinformulierung gD2t (1-1.000 µg), 3D-MPL (10-200 µg) und ein Aluminiumsalz (100-1.000 µg) umfaßt.

11. Verwendung gemäß Anspruch 10, worin die Vakzinformulierung gD2t (20 µg), 3D-MPL (50 µg) und Alaun (500 µg) umfaßt.

12. Verwendung gemäß einem der vorstehenden Ansprüche, wobei die Vakzinformulierung an weibliche Subjekte in Intervallen von 0, 1 und 6 Monaten verabreicht wird oder für die Verabreichung hergestellt wird.

13. Verwendung gemäß einem der vorstehenden Ansprüche, worin die Vakzinformulierung intramuskulär verabreicht wird.

## Revendications

1. Utilisation d'une glycoprotéine HSV D ou d'un fragment immunologique de celle-ci et d'un adjuvant dans la préparation d'un vaccin pour l'administration à un sujet femelle humain HSV1-/2 pour la prévention de la maladie de l'herpès génital.

2. Utilisation selon la revendication 1, dans laquelle l'adjuvant est un adjuvant induisant un TH-1.

3. Utilisation selon les revendications 1 ou 2, dans laquelle la glycoprotéine HSV D est une glycoprotéine tronquée.

4. Utilisation selon la revendication 3, dans laquelle la glycoprotéine tronquée est une HSV gD2 et est dépourvue de la zone de fixation à terminal C (gD2t).

5. Utilisation selon l'une quelconque des revendications précédentes, dans laquelle le vaccin comprend en outre un antigène dérivé de HPV.

6. Utilisation selon l'une quelconque des revendications précédentes, dans laquelle l'antigène ou la combinaison d'antigènes est formulé(e) avec un excipient adapté.

7. Utilisation selon la revendication 6, dans laquelle l'excipient est un hydroxyde d'aluminium (alun), un phosphate d'aluminium ou une émulsion huile en eau.

8. Utilisation selon l'une quelconque des revendications précédentes, dans laquelle l'adjuvant est l'adjuvant induisant un TH-1 3D-MPL.

9. Utilisation selon la revendication 8, dans laquelle les particules de 3D-MPL sont suffisamment petites pour être filtrées de façon stérile, à travers une membrane à 0,22 micron.

10. Utilisation selon l'une quelconque des revendications précédentes, dans laquelle la formulation de vaccin comprend du gD2t (1-1 000 µg), 3D-MPL (10-200 µg) et un sel d'aluminium (100-1 000 µg).

11. Utilisation selon la revendication 10, dans laquelle la formulation de vaccin comprend du gD2t (20 µg), du 3D-MPL (50 µg) et de l'alun (500 µg).

12. Utilisation selon l'une quelconque des revendications précédentes, dans laquelle la formulation de vaccin est administrée à, ou fabriquée pour administration à des sujets féminins à des intervalles de 0, 1 et 6 mois.

13. Utilisation selon l'une quelconque des revendications précédentes, dans laquelle la formulation de vaccin est administrée par voie intramusculaire.
